# FASCICULE DE BREVET EUROPEEN

(11) **EP 1 448 791 B1**
(45) Date de publication et mention de la délivrance du brevet: **09.08.2006**
(21) Numéro de dépôt: 02799760.0
(22) Date de dépôt: 28.11.2002
(51) Int. Cl.: C12Q 1/34, C12Q 1/44

(54) **METHODE DE DETECTION D'UNE ACTIVITE CATALYTIQUE**
VERFAHREN ZUM FESTSTELLEN DER KATALYTISCHEN AKTIVITÄT
METHOD FOR DETECTING CATALYTIC ACTIVITY

(30) Priorité: 28.11.2001 FR 0115389
(43) Date de publication de la demande: 25.08.2004
(73) Titulaire: Proteus, 30000 Nîmes (FR); Université de Berne, 3012 Berne (CH)
(72) Inventeur: REYMOND, Jean-Louis, CH-1630 Bulle (CH); WAHLER, Denis, F-30900 Nîmes (FR)
(74) Mandataire: Breese, Pierre
(86) Numéro de dépôt international: PCT/FR2002/004097
(87) Numéro de publication internationale: WO 2003/046211

(56) Documents cités:
- EP-A- 0 317 243
- EP-A- 0 810 290
- WO-A-01/36662
- BADALASSI F. ET AL.: "A versatile periodate-coupled fluorogenic assay for hydrolytic enzymes" ANGEWANDTE CHEMIE INTERNATIONAL EDITION, vol. 39, no. 22, 17 novembre 2000 (2000-11-17), pages 4067-4070,

## Description

La présente invention concerne une méthode de détection d'une activité catalytique d'un échantillon.

Pour développer de nouveaux procédés de biocatalyse utiles notamment à l'industrie chimique et pharmaceutique et à l'ingénierie des protéines, il est nécessaire de disposer de méthodes de détection d'activités catalytiques fiables, rapides et peu onéreuses.

Il existe dans l'art antérieur deux types de substrats permettant la détection d'une activité catalytique.

On connaît d'une part les substrats du type modifiés par un groupement détectable capables de libérer un signal après leur transformation par une activité catalytique particulière et d'autre part les techniques utilisant les substrats du type naturels comme indiqué ci-après.

Parmi les substrats modifiés, il est possible d'utiliser des substrats de type ester d'alcool aromatique qui libèrent après leur transformation un alcool aromatique coloré ou fluorescent (D. C. Demirjian *et al*. Top. Curr. Chem. 1999, 200, 1). Ces types de substrat ont un désavantage majeur, car la molécule chromogène ou fluorogène est un groupe fortement activé, ce qui rend ces substrats instables. Les réactions de détection qui en découlent peuvent en conséquence être non-spécifiques.

Une deuxième classe de substrats modifiés conduit à la révélation d'un produit obtenu après réaction secondaire enzymatique et/ou spontanée (N. Jourdain *et al*. Tetrahedron Lett. 1998, 39, 9415 ; K.L. Matta *et al*., Carbohydr.Res. 1981, 90, C1-C3 ; G.Klein et J.-L. Reymond, Helv.Chim.Acta 1999, 82, 400). Cette deuxième classe de substrats est plus stable. Cependant, cette deuxième classe de substrats est limitée à des utilisations particulières. En effet, le dosage de la réaction se fait directement sur le produit libéré en utilisant une enzyme. Le produit libéré doit donc répondre à des caractéristiques structurales bien précises, qui limite de ce fait la diversité des activités catalytiques pouvant être détectée.

Une troisième classe de substrats modifiés a été mise au point pour détecter des activités catalytiques en évitant les problèmes soulevés préalablement (Badalassi F. et al. Angew Chem Int Ed Engl. 2000, 39(22):4067-4070). Mais ces substrats correspondent toujours à des substrats modifiés.

D'autre part, on connaît les substrats naturels capables de révéler une activité catalytique. Cependant, les différentes techniques utilisant des substrats naturels sont soit complexes et lourdes à mettre en oeuvre pour du criblage à haut débit (instrumentation lourde dans le cas de la thermographie IR, la CE, HPLC, GC, MS), soit limitées à des activités catalytiques particulières susceptibles par exemple d'induire une différence de pH et/ou à des conditions réactionnelles étroitement définies (mesure des variations de pH, enzymes secondaires). La plupart de ces mesures sont également très chères à réaliser à cause du coût soit de l'instrumentation, soit des réactifs mis en jeu, en particulier les enzymes secondaires et anticorps anti-produit (M. T. Reetz *et al*. Angew. Chem. 1999, 111, 1872 ; A. Holzwarth *et al*. Angew. Chem. 1998, 110, 2788 ; M. T. Reetz *et al*. Angew. Chem. 2000, 112, 1294, Angew. Chem. Int. Ed. Engl. 2000, 39, 1236 ; Taran *et al*. Tetrahedron Lett. 1999, 40, 1887, 1891 ; S. M. Firestine *et al*. Nat. Biotechnol 2000, 18, 544 ; F. Moris-Varas *et al.* Bioorg. Med. Chem. 1999, 7, 2183).

La présente invention vise à offrir une méthode de détection d'une activité catalytique qui s'affranchit des différents problèmes techniques cités ci-dessus et qui permet l'utilisation de tous types de substrats.

Ce but est atteint grâce à une méthode de détection d'une activité catalytique d'un échantillon comprenant :
- la mise en contact d'un substrat (S) avec l'échantillon susceptible de posséder l'activité catalytique que l'on souhaite détecter,
- l'ajout d'un réactif (X) capable de réagir soit avec un groupement chimique du substrat (S) soit avec un groupement chimique du produit (P) formé,
- l'ajout d'un révélateur (R) capable de réagir avec le réactif (X),
- la détection de la transformation du révélateur (R), et caractérisée en ce que la quantité d'équivalents de réactifs (X) est inférieure à la somme de la quantité d'équivalents de (S) non consommé ou de (P) formé, et d'équivalents de révélateurs (R).

Le réactif (X) est ajouté après un temps d'incubation entre le substrat (S) et l'échantillon. Le réactif (X) réagit après un temps d'incubation donné avec soit le substrat (S) non consommé par l'activité catalytique de l'échantillon soit le produit (P) formé. L'activité catalytique peut ainsi être mesurée au cours du temps.

On peut distinguer deux formes de réalisation de la méthode de l'invention :
- une première forme de réalisation où le réactif (X) est modifié par le substrat (S) et pas par le produit (P),
- une seconde forme de réalisation où le réactif (X) est modifié par le produit (P) et pas par le substrat (S).

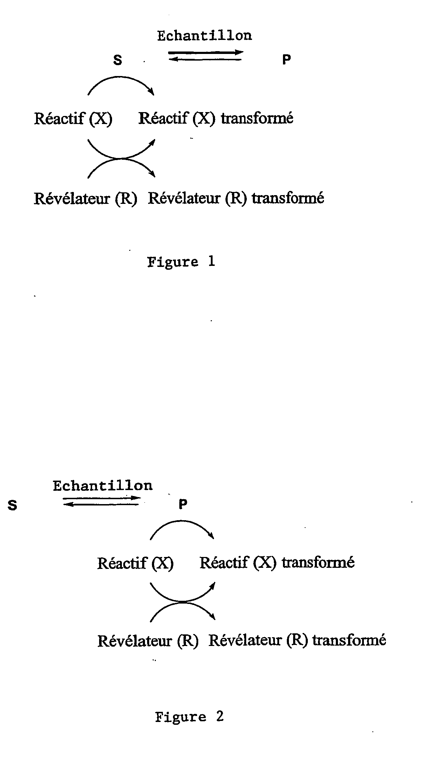

Les schémas des figures 1 et 2 illustrent la méthode de l'invention selon respectivement que (S) ou (P) soit capable de réagir avec le réactif (X).

La méthode de l'invention est basée sur la mise en oeuvre du trio : (S) ou (P) / (X) / (R), où le réactif (X) est capable de réagir avec (S) ou (P) et le révélateur (R).

La méthode de l'invention est basée sur une cascade de réactions où la quantité d'équivalents de réactifs (X) est inférieure à la somme de la quantité d'équivalents de (S) non consommé ou de (P) formé et d'équivalents de révélateurs (R).

La quantité d'équivalents du révélateur (R) utilisée est avantageusement supérieure ou égale à la quantité d'équivalents de réactifs (X).

A titre d'exemple, une détection de l'activité catalytique peut être réalisée en utilisant une quantité de réactif (X) correspondant à 1 fois la quantité maximale de (P) attendu ou de (S) transformé suivant le cas où le réactif (X) réagit avec soit (P) formé ou (S) non consommé.

L'activité catalytique de l'échantillon détectée selon la méthode de l'invention peut être chimique ou enzymatique. Elle correspond à toute activité capable de transformer un substrat (S) en un produit (P). La transformation de (S) en (P) peut être réalisée par plusieurs réactions séquentielles.

L'échantillon susceptible de contenir ladite activité catalytique peut provenir de différentes origines. Il peut être par exemple chimique, biologique, microbiologique, animal, végétal, humain. Il peut provenir de tous types d'environnements et de prélèvements. L'échantillon peut être simple ou complexe, préparé à partir de techniques classiques d'extraction puis éventuellement purifié ou utilisé tel quel.

L'activité catalytique détectée par la méthode de l'invention peut correspondre à une activité nouvelle d'un catalyseur connu. Avantageusement l'activité catalytique correspond à une enzyme. Cette activité peut correspondre à celle d'une enzyme ou d'un mélange d'enzymes.

Cette enzyme est choisie dans le groupe comprenant des hydrolases, des oxydases, des lyases, des ligases, des transférases, des isomérases.

Le substrat (S) peut correspondre à toute molécule pouvant être utilisée pour détecter une activité catalytique selon la méthode de l'invention. Le substrat est donc spécifique de l'activité catalytique recherchée. Il peut correspondre à tous types de substrats comme un substrat naturel ou synthétique pouvant être modifié ou non. Le substrat naturel peut correspondre à titre d'exemple à des huiles végétales pour détecter des activités catalytiques de type lipase.

Le réactif (X) correspond à toute molécule capable de réagir avec un groupement chimique présent soit sur le substrat (S) soit sur le produit (P) mais pas avec les deux.

On entend par groupement chimique tout groupe présent sur le substrat (S) ou le produit (P) et éventuellement sur le révélateur (R) capable de réagir avec le réactif (X). Ils peuvent correspondre à titre d'exemple à des groupes 1,2-diol, 1,2-aminoalcool, 1,2-diamine, alpha-hydroxycétone, alpha-aminocétone, thiol, thioéther, 1,2-catéchol, hydroquinone (=1,4-dihydroxybenzene), pouvant réagir avec le réactif (X) qui peut être un agent oxydant comme le périodate.

Le choix du réactif (X) est fait en fonction de l'activité catalytique que l'on souhaite détecter. Il est choisi de manière à pouvoir réagir, comme on l'a vu précédemment, avec un groupement chimique soit de (P) formé soit de (S) non consommé après un temps d'incubation avec l'éhantillon. Il ne doit pas réagir simultanément avec (S) et (P).

Dans le cas où le réactif (X) réagit avec le produit (P), on peut ajouter le réactif (X) au cours de la transformation catalytique de S en P dans la mesure où le réactif (X) n'affecte pas l'activité catalytique ou la transformation de (S) en (P) et dans la mesure où le réactif (X) résiste aux conditions de transformation de S en P. A titre d'exemple, on évitera d'incuber le réactif correspondant au periodate à très haute température. De plus, si la réaction met en jeu un produit intermédiaire (P') pouvant réagir avec le réactif (X), mais qui peut encore être transformé en produit final (P), il est aussi préférable de ne pas ajouter le réactif avant la fin de la transformation de (S) en (P).

Le révélateur (R) est capable de réagir avec le réactif (X). De plus, la transformation du révélateur (R) par ledit réactif (X) est détectable directement ou indirectement. Enfin, la détection du révélateur (R) est insensible à (S) et (P).

La détection de la transformation du révélateur (R) par le réactif (X) peut correspondre à l'apparition d'un signal ou au contraire à l'extinction d'un signal. Suivant la présence ou l'absence de ce signal, on pourra conclure sur la réalisation de la transformation catalytique de (S) en (P).

Dans une forme particulière, le révélateur (R) peut réagir avec le réactif (X) transformé. Cependant, dans ce cas, le signal mesuré avec le réactif (X) transformé doit être différent de celui mesuré avec le réactif (X).

Le révélateur (R) est choisi de manière à réagir avec le réactif (X) pour donner un composé détectable. A titre d'exemple, le réactif (X) peut modifier un groupement chimique du révélateur (R) pour donner un composé détectable ou le révélateur (R) peut former avec le réactif (X) un complexe non covalent détectable pouvant être notamment chromogénique.

Dans le cas où le révélateur (R) possède un groupement chimique capable de réagir avec le réactif (X), le groupement chimique du révélateur (R) est transformé par le réactif (X).

Dans le cas où la révélation met en oeuvre un groupement chimique du révélateur (R), les groupements chimiques de (S) ou (P) et du révélateur (R) capables de réagir avec le réactif (X) peuvent être différents ou identiques.

Le révélateur (R) peut correspondre à une molécule pouvant être le substrat d'une réaction catalytique ou d'une série de réactions catalytiques différentes de celle(s) capable(s) de transformer (S) en (P).

Dans une forme de mise en oeuvre spécifique de l'invention, le trio suivant est utilisé : (réactif (X) = oxydant chimique / (S) ou (P) et révélateur (R) pouvant posséder les groupements chimiques suivants : 1,2-diol ou 1,2-aminoalcool ou 1,2-diamine ou alpha-hydroxycétone ou alpha-aminocétone ou thiol, thioéther, ou catéchol, ou catécholamine, hydroquinone).

Le substrat (S) est choisi de manière à réagir avec l'échantillon susceptible de contenir l'activité catalytique que l'on souhaite détecter. Dans cette forme de mise en oeuvre, le substrat (S) ou le produit (P) correspondent à un composé de formule (I) suivante : 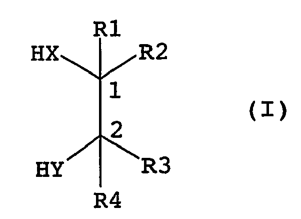 dans laquelle la liaison C1-C2 est sensible à une coupure par une réaction d'oxydation chimique.
- R1 à R4, identiques ou différents, correspondent à un atome d'hydrogène, un groupe alkyle substitué ou non, un groupe fonctionnel substitué ou non.
- X et Y, identiques ou différents, sont choisis parmi un atome d'oxygène, un atome de soufre, une amine de formule -NR8R9, R8 est choisi parmi: un atome d'hydrogène, un groupe alkyle, un groupe aryle, substitués ou non, et R9 n'est pas un atome d'hydrogène.

On entend par groupe fonctionnel tout groupe chimique appartenant à une classe de composés organiques caractérisée par des propriétés chimiques. On peut citer à titre d'exemple de groupe fonctionnel : des amides, des acyles, des alcoxy, des nitriles, des aryles, des hétéroaryles, des alkenyles, des carbonyles, des thiocarbonyles, des carboxyles, des thiocarboxyles, des carbamyles, des thiocarbamyles, des thiocarbamides, des alcools, des thiols, des amines substitués ou non.

Dans une forme préférée, le produit (P) répond à la formule (I) ci-dessus.

Le réactif (X) est choisi de manière à pouvoir réagir avec (S) ou (P) de formule (I). Dans une forme de mise en oeuvre particulière, le réactif (X) correspond à un oxydant chimique capable de cliver la liaison C1-C2 de (S) ou (P) de formule (I).

L'oxydant chimique peut correspondre de manière avantageuse et de façon non limitative à un ou plusieurs des réactifs suivants H5IO6, RuO2, OsO4, (CH3CH2CH2)4N(RuO4), NaClO4, NaIO4, Na3H2IO6, NaMnO4, K2OsO4, KIO4, KMnO4, KRuO4, K2RuO4, LiOCl, l'acétate de plomb, le tétrapropyle ammonium periodate, l'acide chromique ou des sels de celui-ci,NaBiO3,Ph3BiCO3, Ca(OCl)2, les réactifs Ce(IV), Cr(VI), les sels de Co (II), IOAc, I(OAc)3, N-iodosuccinimide, VO(acac), Pb(OAc)4, MnO2, H2O2 ou le mélange des réactifs [H2O2, Na2WO4, H3PO4].

Tout préférentiellement l'agent chimique oxydant est un sel de périodate.

A titre d'exemple, on peut citer les trois formes de révélation décrites ci-dessous.

Dans une forme de mise en oeuvre, le révélateur (R) est choisi parmi l'aminoalcool nitrophénol et les catéchols de formules suivantes :

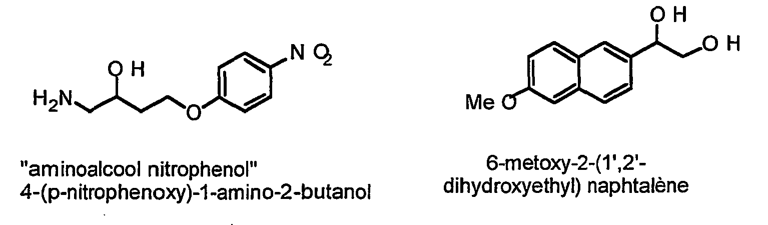

Parmi les révélateurs (R) du periodate n'ayant pas réagit avec S ou P, on peut citer notamment l'aminoalcool nitrophénol et les catéchols.

Les catéchols (1,2-dihydroxybenzene) donnent de manière générale des réactions avec le periodate résultant en un changement de densité optique, et parfois des changement de fluorescence. L'adrénaline et la noradrénaline sont des révélateurs préférés de cette forme de mise en oeuvre. Ils donnent une couleur rouge dans un temps extrêmement court en réagissant avec le periodate. Il n'est pas évident pour l'homme du métier que l'adrénaline puisse servir de révélateur quantitatif du periodate. En effet, les quinones produits de l'oxydation se décomposent au cours du temps pour former des polymères. De plus, cette révélation met en oeuvre une double oxydation. Les exemples de la littérature (El-Kommos *et al*.(1990). J. Assoc. Off. Anal. Chem., 73, 516-520) ne renseignent pas sur le fait que la deuxième oxydation est visiblement plus rapide que la première, ce qui permet une conversion de l'adrénaline en adrénochrome même avec peu de periodate par rapport à l'adrénaline. De ce fait, il n'est absolument pas évident d'utiliser le trio (Réactif = périodate / S ou P = composé de formule I / Révélateur = catéchol) pour détecter une activité catalytique selon la méthode de l'invention.

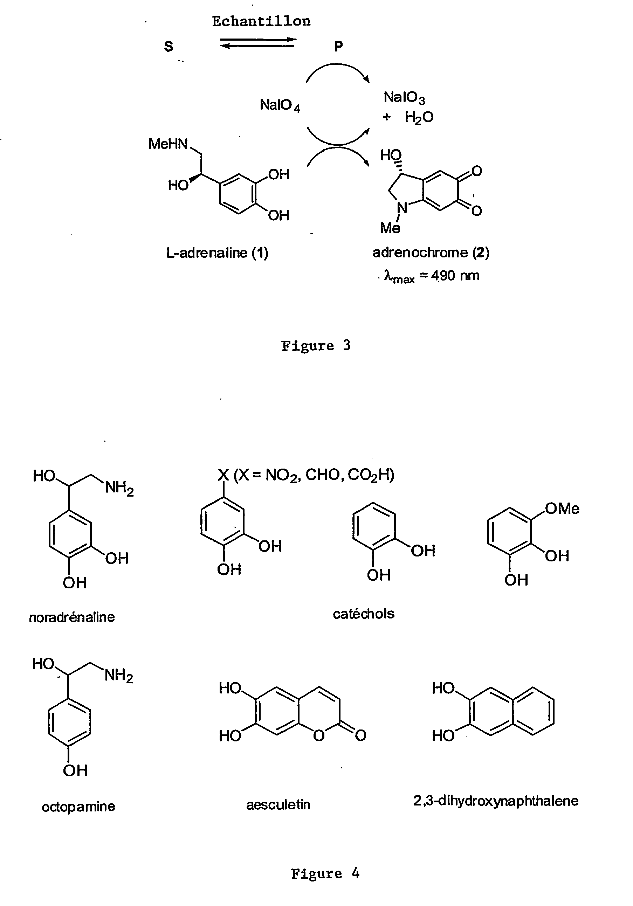

Ce principe de révélation d'activité catalytique utilisant le périodate comme réactif (X) et l'adrénaline comme révélateur (R) est illustré à la figure 3.

L'octopamine peut également être utilisé comme révélateur du périodate, dont l'oxydation de la fonction 1,2-aminoalcool libère le p-hydroxybenzaldéhyde, qui montre une absorbance forte à 330 nm (UV proche, facilement détectable).

La figure 4 donne des exemples de révélateur (R) de NaIO₄ de type catéchol.

Dans une autre forme de mise en oeuvre, le révélateur (R) correspond à un composé de formule (I) pouvant être détecté après avoir réagi avec le réactif (X).

Le révélateur correspondant à un composé de formule (I), décrit précédemment, présente la particularité de pouvoir être détecté après avoir réagi avec le réactif (X) non transformé par (S) non consommé ou (P) formé après un temps d'incubation avec l'échantillon.

Selon un premier mode de mise en oeuvre de révélation directe, le révélateur (R) répondant à la formule (I) est oxydé en présence du réactif (X), selon le schéma réactionnel suivant :

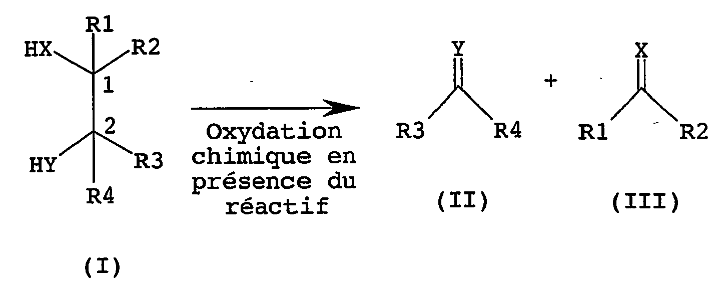

Les propriétés des produits de formules (II) et/ou (III) libérés peuvent alors être directement détectées.

A titre d'exemples non limitatifs on peut citer parmi ces propriétés des composés de formules (II) et/ou (III), une propriété physique, telle que la solubilité, une propriété physico-chimique, telle qu'une propriété spectrale, ou une propriété biologique, telle que l'activation d'une enzyme, une odeur, ou l'action d'une phéromone.

Les composés de formules (II) ou (III) peuvent correspondre à des cétones aromatiques, par exemple une cétone béta-aromatique que l'on détecte par une variation spectrale, un aldéhyde comme le benzaldéhyde ou le citronellal que l'on détecte par l'odeur ou à une phéromone que l'on détecte par l'attraction d'insectes. (Suzuki et al., (1980), Agric.Biol.Chem. 44, 2519 ; Millar et al. (1996), Bioorg.Med.Chem. 3, 331-340).

Dans un deuxième mode de révélation indirecte, au moins un des groupes R1 à R4 du composé de formule (I) répond à la formule (IV) suivante : 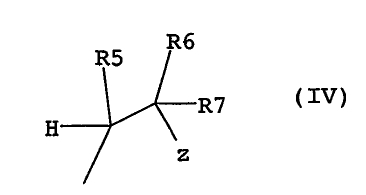 dans laquelle :
- R5, R6 et R7, identiques ou différents, représentent un atome d'hydrogène, un groupe alkyle substitué ou non ou un groupe fonctionnel substitué ou non, et
- Z est un précurseur d'un produit détectable ZH.

Le révélateur (R) de formules (I), en présence du réactif, est oxydé pour libérer à titre d'exemple les composés de formules (V) et (III). Le composé de formule (V) subit une réaction de béta-elimination avantageusement spontanée conduisant au produit détectable ZH, selon le schéma réactionnel suivant :

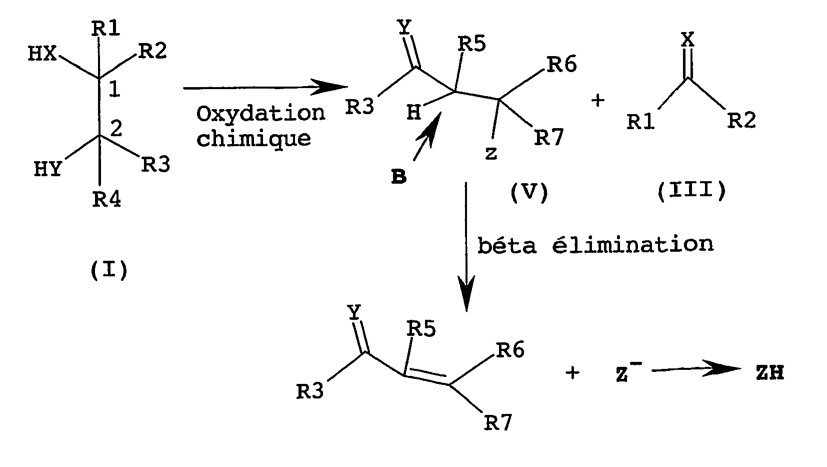

Cette réaction de béta-élimination avantageusement spontanée, est réalisée préférentiellement en présence d'une base désignée « B » dans le schéma ci-dessus qui peut correspondre à l'albumine de sérum de boeuf (BSA).

Parmi les propriétés du composé ZH, on peut citer à titre d'exemples non limitatifs une propriété physique telle que la solubilité, une propriété physico-chimique telle qu'une propriété spectrale ou une propriété biologique telle que l'induction de croissance de bactéries.

Le composé ZH est choisi parmi un alcool aromatique, un alcool hétéroaromatique, une amine hétéroaromatique, un atome d'halogène, ou un ester phosphorique. A titre d'exemples non limitatifs peuvent être cités, la fluorescéine, la phenolphtaléine, le rouge de phénol, le p-nitrophénol l'o-nitrophénol, le 2,4-dinitrophénol, l'acide 6-hydroxynaphtoïque, l'acide 8-hydroxy-pyrène 1,3,6-trisulfonique, la tyrosine, la luciférine, l'indolyle, le 5-bromo-4-chloro-indolyle, le quinolinium, le nitro-anilinium, ou la pyridoxamine.

Dans un troisième mode de révélation, le révélateur (R) répond à la formule (I) dans laquelle :
- un des R1 à R2 et un des R3 à R4 ont la même signification que précédemment,
- les autres R1 ou R2 et R3 ou R4 interagissent entre eux.

Dans ce cas, la coupure de la liaison C1-C2 du révélateur de formule (I), mis en présence du réactif, provoque une variation spectrale détectable.

Un exemple non limitatif d'interaction entre les R1 ou R2 et R3 ou R4 est un transfert d'énergie de type FRET (Fluorescent Resonance Energy Transfert).

Enfin, le révélateur peut également correspondre au précurseur d'un inhibiteur d'un catalyseur.

En présence du réactif (X), le précurseur de l'inhibiteur est transformé en un inhibiteur d'un catalyseur d'une réaction détectable. Ce catalyseur est différent de l'activité catalytique que l'on souhaite détecter avec le procédé de l'invention. L'inhibiteur peut correspondre à titre d'exemple au phénol, le précurseur correspondant peut correspondre notamment à un composé de formule (VI) suivante :

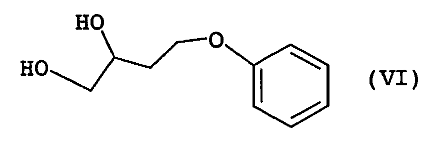

Ce trio (réactif (X) = oxydant chimique / S ou P et révélateur (R) dont les groupements chimiques correspondent à 1,2-diol ou 1,2-aminoalcool ou 1,2-diamine ou alpha-hydroxycétone ou alpha-aminocétone ou thiol, thioéther, cathecol ou cathecolamine ou hydroquinone) permet à titre d'exemple de détecter selon le procédé de l'invention des phytases, lipases, epoxide hydrolases, amidases, des acylases ou des estérases.

Dans une autre forme de mise en oeuvre de l'invention, le trio suivant est utilisé : (réactif (X) = Dansyl-hydrazine / (S) ou (P) et révélateur (R) dont les groupements chimiques correspondent à des cétones ou aldéhydes ou hémiacétals de type alcool).

Le substrat (S) est toujours capable de réagir avec l'activité catalytique que l'on souhaite détecter.

Dans une autre forme de mise en oeuvre, le substrat (S) ou le produit (P) correspond à des aldéhydes ou des cétones O=C(molécule). La détection de la transformation catalytique se fait donc à titre d'exemple de la façon suivante. On utilise comme réactif (X) une hydrazine fluorescente F-NHNH2 (F correspondant à un fluorophore), qui forme le produit fluorescent F-NH-N=C(molécule). Ensuite, le réactif F-NHNH2, n'ayant pas réagit avec S ou P, est dosé avec un révélateur (R) correspondant à un aldéhyde ou une cétone quencheur Q-C=O (Q correspondant au quencher), qui forme le couple non-fluorescent Q-CH=N-NH-F.

On peut concevoir une méthode de détection d'une activité catalytique équivalente capable de transformer un substrat (S) en un produit (P) où (S) ou (P) possèdent les groupements chimiques suivants : des aldéhydes ou des cétones O=C(molécule) ou des hémiacétals de type sucre et :
1) le réactif (X) est une hydrazine quencheuse (ex: 4-carboxy-phenyl-hydrazine) de type Q-NH-NH2, et le révélateur (R) un aldéhyde ou cétone fluorescent F-CHO (ex: 6-méthoxynaphthaldéhyde). La réaction entre le réactif (X) non consommé par (S) ou (P) et le révélateur (R) correspond à une extinction de fluorescence.
2) le réactif (X) est une hydrazine fluorescente F1-NHNH2 et le révélateur (R) un aldéhyde ou cétone F2-CHO. La réaction entre le réactif (X) non consommé par (S) ou (P) et le révélateur (R) correspond à un phénomène de type FRET entre F1 et F2 pouvant être observé. A titre d'exemple F1 = fluoresceine thiosemicarbazide et F2 = rhodamine aldéhyde.

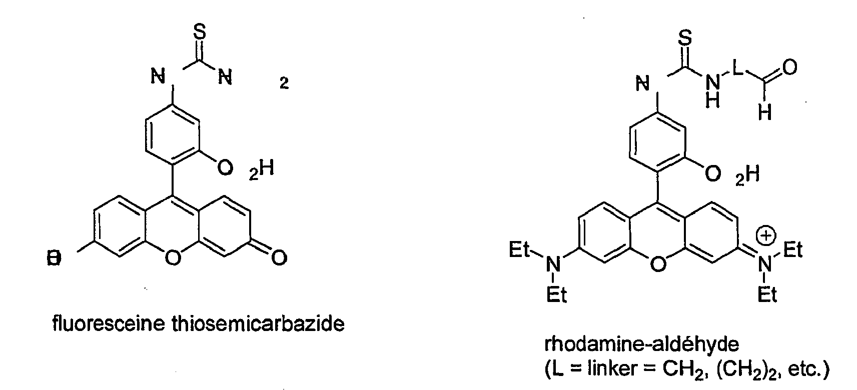

Ce type de trio (réactif (X) = Dansyl-hydrazine / (S) ou (P) et révélateur (R) dont les groupements chimiques correspondent à des cétone ou aldéhyde ou hémiacétal de type sucre) permet à titre d'exemple de détecter selon le procédé de l'invention l'oxydation ou la réduction de cétones et d'aldéhydes, la réaction oxy-cope, la coupure oxidative de double liaisons et de diols, la dimérisation réductive d'aldéhyde ou cétone, l'amination réductive d'aldéhyde ou cétone, les additions de nucléophiles divers sur les cétones et aldéhydes (cyanure, bisulfite, etc.), les réactions d'aldolisation et de rétroaldolisation.

D'autres trios peuvent être envisagés pour réaliser la méthode de l'invention. On peut encore citer les trios suivant :
- (S ou P dont les groupements chimiques correspondent aux aldéhyde, cétone, ou hémiacétal type sucre / X = NaCN, NaBH4, NaHSO3 / R = 6-methoxynaphthaldéhyde) où le révélateur (R) ayant réagi avec le réactif (X) non consommé ou transformé par (S) ou (P) devient non fluorescent.
- (S ou P dont le groupement chimique correspond à RCOOH (acide carboxylique) / X = NaOH / R = 4-nitrophenol) où le révélateur (R) ayant réagi avec le réactif (X) non consommé ou transformé par (S) ou (P) induit un changement de pH.

La méthode de l'invention présente une grande souplesse. Elle peut être mise en oeuvre sur n'importe quel type de substrat. En effet, il est possible notamment de mesurer l'activité de lipases sur des huiles à n'importe quel pH entre 2 et 10, avec ou sans cosolvants, de manière colorimétrique.

La méthode de détection d'activités catalytiques de la présente invention permet de s'affranchir complètement de la structure des substrats, on peut utiliser tout type de substrats spécifiques naturels ou modifiés d'une activité catalytique donnée. A titre d'exemple, on peut citer l'utilisation de précurseur de diol ou hydroxycétones, en particulier des substrats naturels, comme démontré ici avec l'utilisation d'huile végétale comme substrat ou encore le criblage de la condensation de type benzoine catalysée par la pyruvate décarboxylase, qui donne une hydroxycétone à partir de benzaldéhyde et de pyruvate.

La méthode de l'invention permet de mesurer une activité catalytique sur une grande diversité de substrats. On peut citer par exemple l'activité des époxydes hydrolases sur une grande diversité de substrats, ce qui n'est pas possible autrement de manière colorimétrique simple. En effet il faudrait sinon déterminer la formation de chaque produit séparément par HPLC, GC ou autre méthode analytique lourde.

En outre, avec la méthode de l'invention, il est possible de suivre des réactions ou le diol (aminoalcool ou diamine) est utilisé comme substrat et disparaît au cours de la réaction.

Dans le cadre de détection à haut débit, la méthode de l'invention permet d'utiliser des substrats commerciaux, ce qui permet d'avoir rapidement une classe étendue de substrats. Ceci est bien illustré dans les exemples ci-dessous.

La méthode de l'invention peut également être utilisée pour détecter la transformation d'un substrat (S) en un produit (P) par une activité catalytique.

L'invention se rapporte donc également à l'utilisation d'une méthode de détection d'une activité catalytique d'un échantillon décrite ci-dessus pour la détection d'une transformation catalytique d'un substrat (S) en un produit (P).

D'autres avantages et caractéristiques de l'invention apparaîtront dans les exemples qui suivent où il sera fait référence aux dessins en annexe dans lesquels :
La figure 1 est un schéma de la méthode de l'invention où le réactif (X) réagit avec le substrat (S).
La figure 2 est un schéma de la méthode de l'invention où le réactif (X) réagit avec le produit (P).
La figure 3 illustre le principe de la méthode de l'invention utilisant le périodate comme réactif (X) et l'adrénaline comme révélateur (R).
La figure 4 représente des exemples de révélateur (R) de NaIO₄ de type catéchol.
La figure 5 montre les substrats d'enzymes utilisés dans les exemples suivants.
Les figures 6 et 7 représentent la détection d'activités lipase et estérase par la méthode de l'invention respectivement en utilisant comme révélateur le nitrophenolaminoalcool et l'adrénaline.
Les figures 8 et 9 représentent les résultats de détection de phytase respectivement :
   - à différentes températures selon la méthode de l'invention en utilisant comme révélateur le nitrophenolaminoalcool.
   - à différents pH en utilisant comme révélateur l'adrénaline.
Les figures 10 et 11 représentent les résultats de détection d'activités époxyde hydrolase selon la méthode de l'invention respectivement en utilisant comme révélateur le nitrophenolaminoalcool et l'adrénaline.
La figure 12 donne un exemple sur plaque de détection de l'Epoxyde hydrolase d'*Aspergillus Niger.*

### EXEMPLE 1 : DETECTION D'ACTIVITES LIPASE ET ESTERASE.

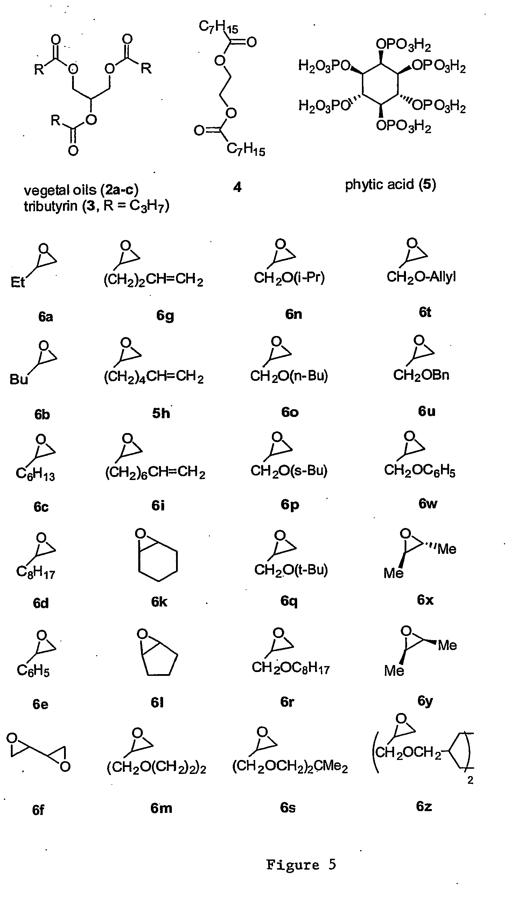

Les réactions de détection sont effectuées à l'aide de 5 substrats décrits à la figure 5. Trois concentrations d'activités enzymatiques sont testées pour effectuer la détection.

### 1) Première forme de mise en oeuvre.

i) Les enzymes diluées dans un tampon phosphate 20 mM pH 7.2 sont ajoutés à des substrats correspondant à des huiles végétales **2a-c** (0,05 mL dans 0,4 mL de tampon dans un eppendorf de 1.5 mL, agitateur à 1200 tour/min.) ou substrats **3** ou **4** (10 mM) à 26°C pendant 60 min. Ces différents substrats sont présents sur la figure 5.
ii) Ajout au niveau de la réaction catalytique précédente durant 30 min d'un réactif correspondant à 1 mM NaIO₄ capable de réagir chimiquement avec le produit P.
iii) Ajout de 1,5 mM du révélateur NH2CH₂CHOHCH₂CH₂OC₆H₄NO₂ (= nitrophenol aminoalcool) + 2 mg.mL⁻¹ BSA, 60 min.

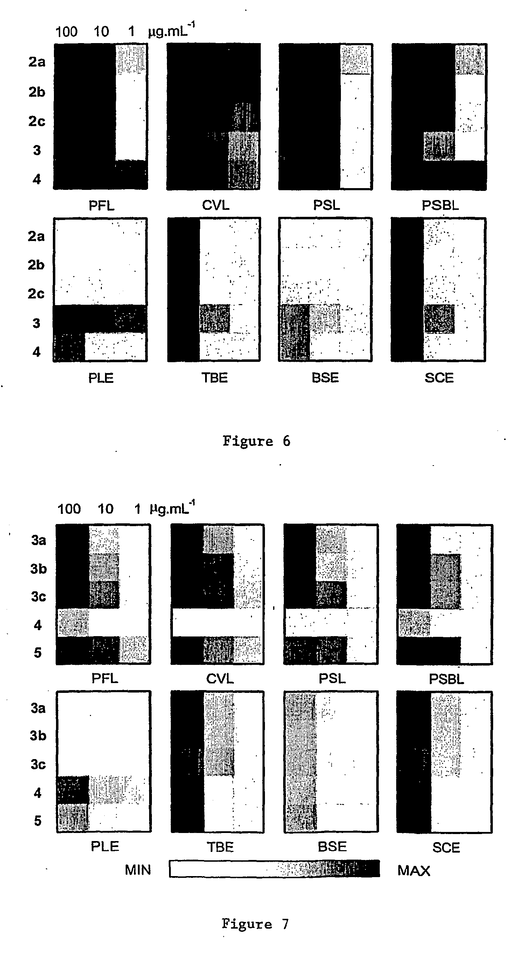

La figure 6 en annexe rapporte les résultats de détection d'activités lipase et estérase avec des huiles végétales **(2a =** huile d'olive, **2b** = huile de tournesol, 2c = huile de pépins de raisin), tributyrine **(3)** et ethylene glycol bis-octanoate **(4).** Les zones colorés vont du blanc (pas d'activité, aucune réduction de la couleur du nitrophénol) à noir (aucune couleur restante du nitrophenol, activité maximum). Abbréviations des échantillons testés (Fluka): PFL = *Pseudomonas fluorescens* lipase (F62321); CVL *= Chromobacterium viscosum* lipoprotein lipase (F62333); PSL = *Pseudomonas* sp. lipoprotein lipase (F62335); PSBL = *Pseudomonas* sp. Type B lipoprotein lipase (F62336); PLE = pig liver esterase (F46058); TBE = *Thermoanaerobium brockii* esterase (F46061); BSE = *Bacillus* sp. esterase (F46062); SCE = *Saccharomyces cerevisiae* esterase (F46071).

### 2) Deuxième forme de mise en oeuvre.

i) Les enzymes diluées dans un tampon phosphate 20 mM pH 7,2 sont ajoutés à des substrats correspondant à des huiles végétales **2a-c** (0,05 mL dans 0,4 mL de tampon dans un eppendorf de 1.5 mL, agitateur à 1200 tour/min.) ou substrats **3** ou **4** (10 mM) à 26°C pendant 30 min avec 1 mM de NaIO₄. Ces différents substrats sont présents sur la figure 5.
ii) Ajout de 1,5 mM d'adrénaline, 5 minutes.

La figure 7 en annexe illustre les résultats de détection d'activité lipase et estérase avec des huiles végétales **(2a =** huile d'olive, **2b** = huile de tournesol, 2c = huile de pépins de raisin), tributyrine **(3)** et ethylene glycol bis-octanoate **(4).** Les zones colorés vont du blanc (pas d'activité, aucune réduction de la couleur de l'adrénochrome) à noir (aucune couleur restante de l'adrénochrome, activité maximum). Abbréviations des échantillons testés (Fluka): PFL *= Pseudomonas fluorescens* lipase (F62321); CVL *= Chromobacterium viscosum* lipoprotein lipase (F62333); PSL *= Pseudomonas* sp. lipoprotein lipase (F62335); PSBL *= Pseudomonas* sp. Type B lipoprotein lipase (F62336); PLE = pig liver esterase (F46058); TBE = *Thermoanaerobium brockii* esterase (F46061); BSE *= Bacillus* sp. esterase (F46062); SCE *= Saccharomyces cerevisiae* esterase (F46071).

### EXEMPLE 2 : DETECTION D'ACTIVITE PHYTASE.

### 1) Première forme de mise en oeuvre : en fonction de la température.

i) aq. 10 mM phytate, pH 5.0, phytase (0.1 mg.mL⁻¹), 60 min. à la température indiquée;
ii) 1 mM NaIO₄, 30 min., 26 °C;
iii) ajuster le pH à 9.0 avec 0.1 N NaOH, ensuite ajouter 1,5 mM nitrophenolaminoalcool, 2 mg.mL⁻¹ BSA, 60 min.

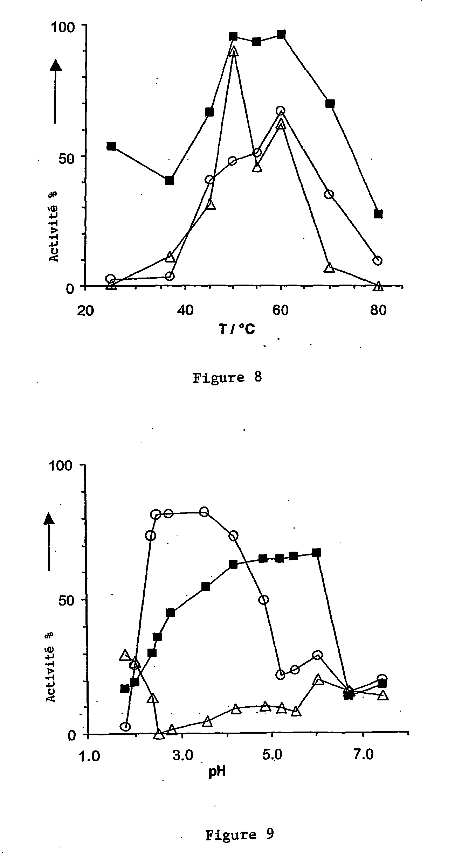

   la figure 8 représente les résultats de détection de phytase à différentes température : (■) Natuphos phytase (+ 1 mM CaCl2), (Δ) Novo phytase (+ 1 mM CaCl2), (o) *Aspergillus ficuum* phytase (1 mM CaCl2 + 100 nM MnCl2). Aucune activité n'est observée sans enzyme.

### 2) Deuxième forme de mise en oeuvre : en fonction du pH.

i) aq. 10 mM phytate, phytase (0.1 mg.mL-1), 55°C, 60 min. au pH indiqué;
ii) 1 mM NaIO4, 30 min., 26 °C;
iii) Ajout de 1.5 mM d'adrénaline, 5 minutes.

La figure 9 représente les résultats sont illustrés sur la détection de phytase à différents pH : (■) Natuphos phytase (+ 1 mM CaCl2), (Δ) Novo phytase (+ 1 mM CaCl2), (o) *Aspergillus ficuum* phytase (1 mM CaCl2 + 100 nM MnC12).

### EXEMPLE 3 : DETECTION D'ACTIVITES EPOXYDE HYDROLASE (EH).

Les réactions de détection sont effectuées à l'aide de grille de substrats époxyde 6a-z disposés selon la figure 5.

### 1) Première forme de mise en oeuvre.

i) 10 mM epoxide dans aq. 20 mM phosphate pH 7.2 avec l'enzyme
ii) ajout de 1 mM NaIO₄, 30 min., 26°C; ajout de 1,5 mM de nitrophenolaminoalcool, 2 mg.mL⁻¹ BSA, 60 min., 26°C.
iii) Aucune activité n'est détectée sans enzyme ou en présence de BSA (2 mg/mL).

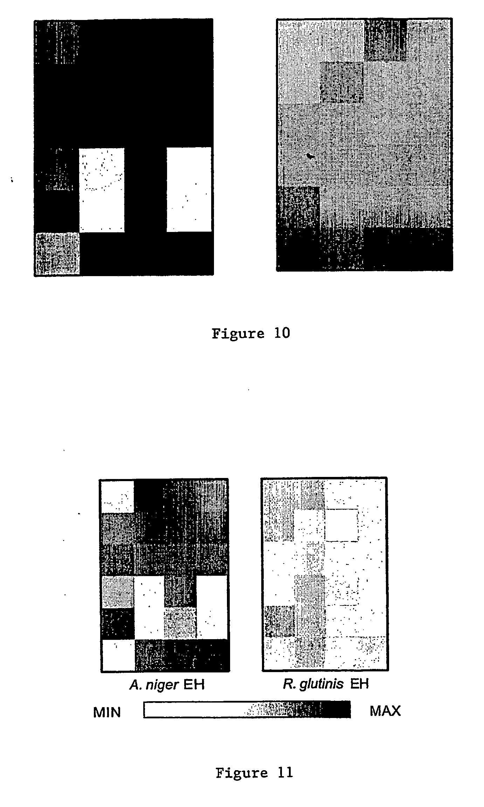

Les différents résultats de détection d'activités époxyde hydrolase sont illustrés sur la figure 10.

### 2) Deuxième forme de mise en oeuvre.

i) 10 mM epoxide dans aq. 20 mM phosphate pH 7.2 avec l'enzyme et 1 mM NaIO₄, 30 min., 26°C avec 0.05 mg/mL aspergillus Niger EH , ou 37°C avec 0.1 mg/mL Rhodotorula glutinis EH.
ii) ajout de 1,5 mM d'adrénaline, 5 min, 26°C.

Les figures 10 et 11 représentent les résultats de détection d'activités époxyde hydrolase :
- Figure 10 : Grille de substrats époxyde 6a-z disposés selon la figure 5. A gauche: *Aspergillus niger* EH (50 µg.mL-1), 60 min., 26°C; A droite: *Rhodoturula glutinis* EH (0.5 mg.mL-1), 37°C, 120 min.

Figure 11 : Grille de substrats époxyde 6a-z disposés selon la figure 5. A gauche: *Aspergillus niger* EH (50 µg.mL-1), 30 min., 26°C; A droite: *Rhodoturula glutinis* EH (0.1 mg.mL-1), 37°C, 30 min.

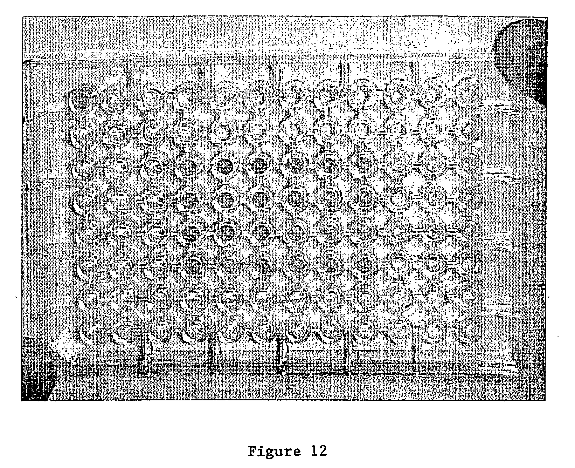

La figure 12 donne un exemple sur plaque de détection de l'Epoxyde Hydrolase (EH) d'*Aspergillus Niger,* réalisé par révélation directe à l'adrénaline.Conditions : pH 7.2, 25 microg/mL enzyme, 10 mM substrats, 1 mM NaIO4, 30 min., puis ajout de 1.5 mM adrénaline, réaction instantanée (<30 sec.). Les puits colorés sont ceux où le periodate n'a pas été consommé par le diol formé par hydrolyses des époxydes, donc où aucune réaction n'a eu lieu (contrôle en haut à gauche).

## Revendications

1. Méthode de détection d'une activité catalytique d'un échantillon, **caractérisée en ce qu'**elle comprend :
- la mise en contact d'un substrat (S) avec l'échantillon susceptible de posséder l'activité catalytique que l'on souhaite détecter,
- l'ajout d'un réactif (X) capable de réagir soit avec un groupement chimique du substrat (S) soit avec un groupement chimique du produit (P) formé,
- l'ajout d'un révélateur (R) capable de réagir avec le réactif (X),
- la détection de la transformation du révélateur (R),
et **caractérisée en ce que** la quantité d'équivalents de réactifs (X) est inférieure à la somme de la quantité d'équivalents de (S) non consommé ou de (P) formé, et d'équivalents de révélateurs (R).

2. Méthode de détection d'une activité catalytique d'un échantillon selon la revendication 1, **caractérisée en ce que** le réactif (X) est modifié par le substrat (S) et pas par le produit (P).

3. Méthode de détection d'une activité catalytique d'un échantillon selon la revendication 1, **caractérisée en ce que** le réactif (X) est modifié par le produit (P) et pas par le substrat (S).

4. Méthode selon l'une des revendications 1 à 3, **caractérisée en ce que** la quantité d'équivalents du révélateur (R) utilisée est supérieure ou égale à la quantité d'équivalents de réactifs (X).

5. Méthode selon les revendications 1 à 4, **caractérisée en ce que** le substrat est un substrat naturel ou synthétique.

6. Méthode selon l'une quelconque des revendications précédentes, **caractérisée en ce que** la détection de la transformation du révélateur (R) par le réactif (X) correspond à l'apparition d'un signal ou à l'extinction d'un signal.

7. Méthode de détection d'une activité catalytique d'un échantillon selon les revendications 1 à 6, **caractérisée en ce que** l'on ajoute le réactif (X) au cours de la transformation du substrat (S) en produit (P).

8. Méthode selon l'une quelconque des revendications précédentes, **caractérisée en ce que** le réactif (X) est un oxydant chimique, (S) ou (P) et révélateur (R) pouvant posséder les groupements chimiques suivants : 1,2-diol ou 1,2-aminoalcool ou 1,2-diamine ou alpha-hydroxycétone ou alpha-aminocétone ou thiol, thioéther, ou catéchol, ou catécholamine, hydroquinone)..

9. Méthode selon la revendication 8,
**caractérisée en ce que** le substrat (S) ou le produit (P) correspondent à un composé de formule (I) suivante : 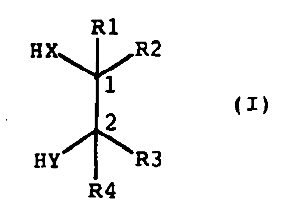 dans laquelle la liaison C1-C2 est sensible à une coupure par une réaction d'oxydation chimique.
- R1 à R4, identiques ou différents, correspondent à un atome d'hydrogène, un groupe alkyle substitué ou non, un groupe fonctionnel substitué ou non.
- X et Y, identiques ou différents, sont choisis parmi un atome d'oxygène, un atome de soufre, une amine de formule -NR8R9, R8 est choisi parmi: un atome d'hydrogène, un groupe alkyle, un groupe aryle, substitués ou non, et R9 n'est pas un atome d'hydrogène.

10. Méthode selon l'une des revendications 8 ou 9, **caractérisée en ce que** le réactif (X) est un sel de périodate.

11. Méthode selon l'une quelconque des revendications 1 à 10, **caractérisée en ce que** le révélateur (R) est choisi parmi l'aminoalcool nitrophénol et les catéchols.

12. Méthode selon l'une quelconque des revendications 1 à 11, **caractérisée en ce que** le révélateur (R) est choisi dans le groupe comprenant l'adrénaline, la noradrénaline et l'octopamine.

13. Méthode selon l'une quelconque des revendications 1 à 10, **caractérisée en ce que** le révélateur (R) de formule (I) est détecté après avoir réagi avec le réactif (X) qui est un oxydant chimique.

14. Méthode selon la revendication 13, **caractérisée en ce que** le révélateur (R) répondant à la formule (I) est oxydé en présence du réactif (X), pour conduire aux produits de formules (II) et (III) : 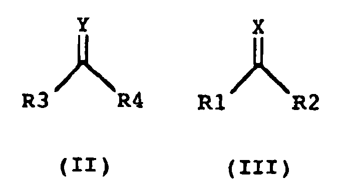 et **en ce que** lesdits produits de formules (II) et/ou (III) libérés sont directement détectées.

15. Méthode selon la revendication 14, **caractérisée en ce que** les composés de formules (II) ou (III) sont choisis dans le groupe comprenant des cétones aromatiques, par exemple une cétone béta-aromatique que l'on détecte par une variation spectrale, un aldéhyde comme le benzaldéhyde ou le citronellal que l'on détecte par l'odeur ou à une phéromone que l'on détecte par l'attraction d'insectes.

16. Méthode selon la revendication 13, **caractérisée en ce que** au moins un des groupes R1 à R4 du composé de formule (I) répondent à la formule (IV) suivante : 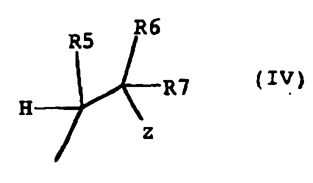 dans laquelle :
- R5, R6 et R7, identiques ou différents, représentent un atome d'hydrogène, un groupe alkyle substitué ou non ou un groupe fonctionnel substitué ou non, et
- Z est un précurseur d'un produit détectable ZH.

17. Méthode selon la revendication 16, **caractérisée en ce que** le révélateur (R) de formules (I), en présence du réactif, est oxydé pour libérer les composés de formules (V) et (III) 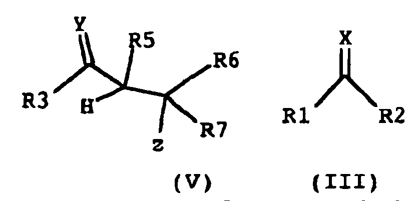 et **en ce que** le composé de formule (V) subit une réaction de béta-élimination avantageusement spontanée conduisant au produit détectable ZH.

18. Méthode selon la revendication 17, **caractérisée en ce que** le composé ZH est choisi dans le groupe comprenant un alcool aromatique, un alcool hétéroaromatique, une amine hétéroaromatique, un atome d'halogène, ou un ester phosphorique.

19. Méthode selon la revendication 13, **caractérisée en ce que** le révélateur (R) répond à la formule (I) dans laquelle :
- un des R1 à R2 et un des R3 à R4 ont la même signification que précédemment,
- les autres R1 ou R2 et R3 ou R4 interagissent entre eux.
et **en ce que** la coupure de la liaison C1-C2 du révélateur (R) de formule (I), en présence du réactif (X), provoque une variation spectrale détectable.

20. Méthode selon la revendication 19, **caractérisé en ce que** l'interaction entre les R1 ou R2 et R3 ou R4 est un transfert d'énergie de type FRET.

21. Méthode selon l'une quelconque des revendications 1 à 10, **caractérisée en ce que** le révélateur (R) est un précurseur d'un inhibiteur d'un catalyseur et **en ce que** en présence du réactif le précurseur de l'inhibiteur est transformé en un inhibiteur d'un catalyseur d'une réaction détectable, ledit catalyseur étant différent de l'activité catalytique que l'on souhaite détecter.

22. Méthode selon l'une des revendications 1 à 8, **caractérisée en ce que** le réactif (X) est la Dansyl-hydrazine, le substrat (S) ou le produit (P) et le révélateur (R) dont les groupements chimiques correspondent à des cétone ou aldéhyde ou hémiacétal de type alcool.

23. Méthode selon la revendication 22, **caractérisée en ce que** le réactif (X) est une hydrazine fluorescente F-NHNH2, où F correspond à un fluorophore qui forme un produit fluorescent F-NH-N=C(molécule), et **en ce que** le réactif (X) F-NHNH2, n'ayant pas réagit avec (S) ou (P) est dosé avec un révélateur (R) correspondant à un aldéhyde ou une cétone quencheur Q-C=O, où Q correspond au quencher, pour former le couple non-fluorescent Q-CH=N-NH-F.

24. Méthode selon la revendication 22, **caractérisée en ce que** le réactif (X) est une hydrazine quencheuse de type Q-NH-NH2, et le révélateur (R) un aldéhyde ou cétone fluorescent F-CHO.

25. Méthode selon la revendication 22, **caractérisée en ce que** le réactif (X) est une hydrazine fluorescent F1-NHNH2 et le révélateur (R) un aldéhyde ou cétone F2-CHO, tel qu'un phénomène de FRET entre F1 et F2 puisse être observé.

26. Utilisation d'une méthode de détection activité catalytique d'un échantillon selon l'une quelconque des revendications précédentes pour la détection d'une transformation catalytique d'un substrat (S) en un produit (P).

## Claims

1. Method of detecting a catalytic activity of a sample, **characterized in that** it comprises:
- contacting a substrate (S) with the sample which may have the catalytic activity to be detected,
- adding a reagent (X) which is capable of reacting either with a chemical group of the substrate (S) or with a chemical group of the product (P) formed,
- adding a developer (R) which is capable of reacting with the reagent (X),
- detecting the conversion of the developer (R),
and **characterized in that** the amount of equivalents of reagent (X) is less than the total of the amount of equivalents of unconsumed (S) or of (P) formed and of equivalents of developers (R).

2. Method of detecting a catalytic activity of a sample according to Claim 1, **characterized in that** the reagent (X) is modified by the substrate (S) and not by the product (P).

3. Method of detecting a catalytic activity of a sample according to Claim 1, **characterized in that** the reagent (X) is modified by the product (P) and not by the substrate (S).

4. Method according to any of Claims 1 to 3, **characterized in that** the amount of equivalents of the developer (R) used is greater than or equal to the amount of equivalents of reagent (X).

5. Method according to Claims 1 to 4, **characterized in that** the substrate is a natural or synthetic substrate.

6. Method according to any one of the preceding claims, **characterized in that** the detection of the conversion of the developer (R) by the reagent (X) corresponds to the appearance of a signal or to the extinction of a signal.

7. Method of detecting a catalytic activity of a sample according to Claims 1 to 6, **characterized in that** the reagent (X) is added during the conversion of the substrate (S) into the product (P).

8. Method according to any one of the preceding claims, **characterized in that** the reagent (X) is a chemical oxidant, where (S) or (P) and developer (R) can have the following chemical groups: 1,2-diol or 1,2-amino-alcohol or 1,2-diamine or alpha-hydroxyketone or alpha-aminoketone or thiol, thioether, or catechol, or catecholamine, hydroquinone.

9. Method according to Claim 8, **characterized in that** the substrate (S) or the product (P) corresponds to a compound of the following formula (I): 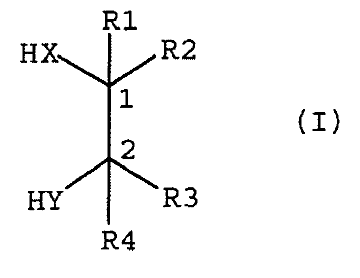 in which the C1-C2-bond is sensitive to cleavage by a chemical oxidation reaction,
- R1 to R4, which are identical or different, correspond to a hydrogen atom, an optionally substituted alkyl group, an optionally substituted functional group,
- X and Y, which are identical or different, are selected among an oxygen atom, a sulphur atom, an amine of the formula -NR8R9, where R8 is selected among a hydrogen atom, an alkyl group, an aryl group, which are optionally substituted, and R9 is other than a hydrogen atom.

10. Method according to Claim 8 or 9, **characterized in that** the reagent (X) is a periodate salt.

11. Method according to any one of Claims 1 to 10, **characterized in that** the developer (R) is selected among the aminoalcohol nitrophenol and the catechols.

12. Method according to any one of Claims 1 to 11, **characterized in that** the developer (R) is selected from the group consisting of adrenalin, noradrenalin and octopamin.

13. Method according to any one of Claims 1 to 10, **characterized in that** the developer (R) of the formula (I) is detected after having reacted with the reagent (X), which is a chemical oxidant.

14. Method according to Claim 13, **characterized in that** the developer (R), which corresponds to formula (I), is oxidized in the presence of reagent (X) to give the products of the formulae (II) and (III): 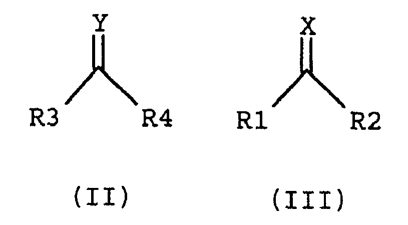 and **in that** these liberated products of formulae (II) and/or (III) are detected directly.

15. Method according to Claim 14, **characterized in that** the compounds of formula (II) or (III) are selected from the group consisting of the aromatic ketones, for example a beta-aromatic ketone, which are detected by spectral variation, an aldehyde, such as benzaldehyde or citronellal, which is detected by the odour, or a pheromone, which is detected by its attracting insects.

16. Method according to Claim 13, **characterized in that** at least one of groups R1 to R4 of the compound of formula (I) corresponds to the following formula (IV): 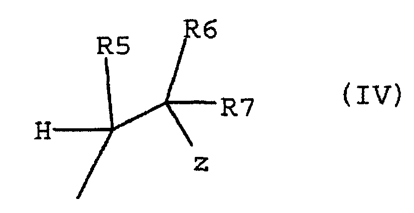 in which:
- R5, R6 and R7, which are identical or different, represent a hydrogen atom, an optionally substituted alkyl group or an optionally substituted functional group, and
- Z is a precursor of a detectable product ZH.

17. Method according to Claim 16, **characterized in that** the developer (R) of the formula (I) is, in the presence of the reagent, oxidized to liberate the compounds of formulae (V) and (III) 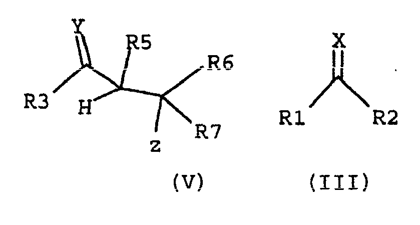 and **in that** the compound of the formula (V) undergoes an advantageously spontaneous beta-elimination reaction which leads to the detectable product ZH.

18. Method according to Claim 17, **characterized in that** the compound ZH is selected from the group consisting of an aromatic alcohol, a heteroaromatic alcohol, a heteroaromatic amine, a halogen atom or a phosphoric ester.

19. Method according to Claim 13, **characterized in that** the developer (R) corresponds to the formula (I), in which:
- one of R1 to R2 and one of R3 to R4 have the same meaning as above,
- the other R1 or R2 and R3 or R4 interact with one another
and **in that** the cleavage of the C1-C2-bond of the developer (R) of the formula (I) in the presence of the reagent (X) causes a detectable spectral variation.

20. Method according to Claim 19, **characterized in that** the interaction between R1 or R2 and R3 or R4 is an FRET-type energy transfer.

21. Method according to any one of Claims 1 to 10, **characterized in that** the developer (R) is a precursor of an inhibitor of a catalyst and **in that** the precursor of the inhibitor is, in the presence of the reagent, converted into an inhibitor of a catalyst of a detectable reaction, said catalyst being different from the catalytic activity to be detected.

22. Method according to any of Claims 1 to 8, **characterized in that** the reagent (X) is dansylhydrazine, the substrate (S) or the product (P) and the developer (R), where the chemical groups correspond to ketone or aldehyde or alcohol-type hemiacetal.

23. Method according to Claim 22, **characterized in that** the reagent (X) is a fluorescent hydrazine F-NHNH₂, where F corresponds to a fluorophor which forms a fluorescent product F-NH-N=C(molecule), and **in that** the reagent (X) F-NHNH₂ which has not reacted with (S) or (P) is assayed with a developer (R) which corresponds to a quench aldehyde or ketone Q-C=O, where Q corresponds to the quencher, to form the non-fluorescent pair Q-CH=N-NH-F.

24. Method according to Claim 22, **characterized in that** the reagent (X) is a quencher hydrazine of the Q-NH-NH₂ type and the developer (R) a fluorescent aldehyde or ketone F-CHO.

25. Method according to Claim 22, **characterized in that** the reagent (X) is a fluorescent hydrazine F1-NHNH₂ and the developer (R) is an aldehyde or ketone F2-CHO, in such a way that the appearance of an FRET between F1 and F2 can be observed.

26. Use of a method of detecting catalytic activity of a sample according to any one of the preceding claims for detecting a catalytic conversion of a substrate (S) into a product (P).

## Patentansprüche

1. Verfahren zum Feststellen einer katalytischen Aktivität einer Probe, **dadurch gekennzeichnet, dass** sie Folgendes umfasst:
- Inkontaktbringen eines Substrats (S) mit der Probe, die möglicherweise die katalytische Aktivität, die es nachzuweisen gilt, aufweist,
- Zugabe eines Reaktanten (X), der fähig ist, mit einer chemischen Gruppe des Substrats (S) oder mit einer chemischen Gruppe des gebildeten Produkts (P) zu reagieren,
- Zugabe eines Entwicklers (R), der fähig ist, mit dem Reaktanten (X) zu reagieren,
- Nachweis der Transformation des Entwicklers (R),
sowie **dadurch gekennzeichnet, dass** die Menge der Reaktantenäquivalente (X) kleiner ist als die Summe der nicht verbrauchten (S)-Äquivalente oder der gebildeten (P)-Äquivalente plus der Entwickleräquivalente (R).

2. Verfahren zum Nachweis einer katalytischen Aktivität einer Probe nach Anspruch 1, **dadurch gekennzeichnet, dass** der Reaktant durch das Substrat (S) und nicht durch das Produkt (P) modifiziert wird.

3. Verfahren zum Nachweis einer katalytischen Aktivität einer Probe nach Anspruch 1, **dadurch gekennzeichnet, dass** der Reaktant (X) durch das Produkt (P) und nicht durch das Substrat (S) modifiziert wird.

4. Verfahren nach einem der Ansprüche 1 bis 3, **dadurch gekennzeichnet, dass** die verwendete Menge an Entwickleräquivalenten (R) gleich oder größer als die Menge der Reaktantenäquivalente (X) ist.

5. Verfahren nach den Ansprüchen 1 bis 4, **dadurch gekennzeichnet, dass** es sich bei dem Substrat um ein natürliches oder synthetisches Substrat handelt.

6. Verfahren nach einem der vorhergehenden Ansprüche, **dadurch gekennzeichnet, dass** der Nachweis der Umwandlung des Entwicklers (R) durch den Reaktanten (X) dem Auftreten eines Signals oder der Extinktion eines Signals entspricht.

7. Verfahren zum Nachweisen einer katalytischen Aktivität einer Probe nach den Ansprüchen 1 bis 6, **dadurch gekennzeichnet, dass** man den Reaktanten (X) während der Umwandlung des Substrats (S) in das Produkt (P) zugibt.

8. Verfahren nach einem der vorhergehenden Ansprüche, **dadurch gekennzeichnet, dass** es sich bei dem Reaktanten (X) um ein chemisches Oxidationsmittel handelt, wobei (S) oder (P) und der Entwickler (R) die folgenden chemischen Gruppen aufweisen können: 1,2-Diol oder 1,2-Aminoalkohol oder 1,2-Diamin oder Alpha-hydroxyketon oder Alpha-aminoketon oder Thiol, Thiolether, oder Katechin, oder Katecholamin, Hydrochinon).

9. Verfahren nach Anspruch 8, **dadurch gekennzeichnet, dass** das Substrat (S) oder das Produkt (P) einer Verbindung der folgenden Formel (I) entsprechen: 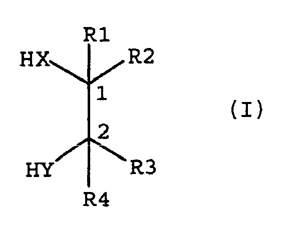 in der die Bindung C1-C2 gegenüber Spaltung durch eine chemische Oxidation empfindlich ist,
- R1 bis R4, die gleich oder verschieden sind, einem Wasserstoffatom, einer gegebenenfalls substituierten Alkylgruppe oder einer gegebenenfalls substituierten funktionellen Gruppe entsprechen,
- X und Y, die gleich oder verschieden sind, aus der Gruppe Sauerstoffatom, Schwefelatom, Amin der Formel -NR8R3, wobei R8 aus der Reihe Wasserstoffatom, Alkylgruppe, Arylgruppe, wobei diese gegebenenfalls substituiert sind, stammt, und R9 nicht ein Wasserstoffatom ist, stammen.

10. Verfahren nach einem der Ansprüche 8 bis 9, **dadurch gekennzeichnet, dass** es sich bei dem Reaktanten (X) um ein Periodatsalz handelt.

11. Verfahren nach einem der Ansprüche 1 bis 10, **dadurch gekennzeichnet, dass** der Entwickler (R) aus der Reihe Nitrophenol-Aminoalkohol sowie Katechine stammt.

12. Verfahren nach einem der Ansprüche 1 bis 11, **dadurch gekennzeichnet, dass** der Entwickler (R) aus der Gruppe Adrenalin, Noradrenalin und Octopamin stammt.

13. Verfahren nach einem der Ansprüche 1 bis 10, **dadurch gekennzeichnet, dass** der Entwickler (R) der Formel (I) nach Reaktion mit dem Reaktanten (X), bei dem es sich um ein chemisches Oxidationsmittel handelt, nachgewiesen wird.

14. Verfahren nach Anspruch 13, **dadurch gekennzeichnet, dass** der Entwickler (R), der der Formel (I) entspricht, in Gegenwart des Reaktanten (X) zu den Produkten der Formeln (II) und (III): 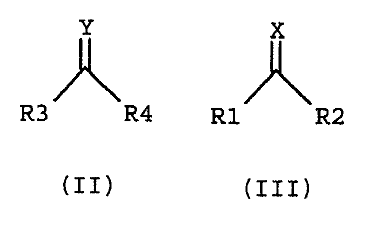 oxidiert wird und **dadurch**, dass diese freigesetzten Produkte der Formeln (II) bzw. (III) direkt nachgewiesen werden.

15. Verfahren nach Anspruch 14, **dadurch gekennzeichnet, dass** die Verbindungen der Formeln (II) oder (III) aus der Gruppe aromatische Ketone, zum Beispiel ein Beta-aromatisches Keton, das auf Grund einer Spektralvariation nachgewiesen wird, ein Aldehyd wie Benzaldehyd oder Citronellal, der bzw. das auf Grund des Geruchs nachgewiesen wird, oder ein Pheromon, das durch Attraktionswirkung auf Insekten nachgewiesen wird, stammen.

16. Verfahren nach Anspruch 13, **dadurch gekennzeichnet, dass** mindestens einer der Gruppen R1 bis R4 der Verbindung der Formel (I) der folgenden Formel (IV) entspricht: 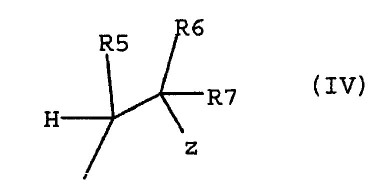 in der:
- R5, R6 und R7, die gleich oder verschieden sind, ein Wasserstoffatom, eine gegebenenfalls substituierte Alkylgruppe oder eine gegebenenfalls substituierte funktionelle Gruppe bedeuten und
- Z eine Vorstufe eines nachweisbaren Produkts ZH ist.

17. Verfahren nach Anspruch 16, **dadurch gekennzeichnet, dass** der Entwickler (R) der Formel (I) in Gegenwart des Reaktanten oxidiert wird, um die Verbindungen der Formeln (V) und (III) 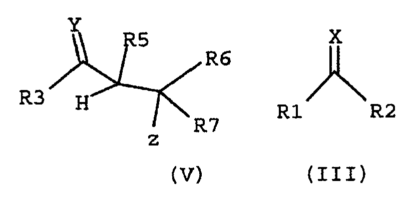 freizusetzen, sowie **dadurch**, dass die Verbindung der Formel (V) eine vorteilhafterweise spontane Beta-Eliminationreaktion durchmacht, die zu dem nachweisbaren Produkt ZH führt.

18. Verfahren nach Anspruch 17, **dadurch gekennzeichnet, dass** die Verbindung ZH aus der Gruppe aromatischer Alkohol, heteroaromatischer Alkohol, heteroaromatisches Amin, Halogenatom oder Phosphorsäureester stammt.

19. Verfahren nach Anspruch 13, **dadurch gekennzeichnet, dass** der Entwickler (R) der Formel (I) entspricht, in der:
- eine der Gruppen R1 bis R2 und eine der Gruppen R3 bis R4 die gleiche Bedeutung wie oben aufweisen und
- die anderen Gruppen R1 oder R2 und R3 oder R4 untereinander wechselwirken
- sowie **dadurch**, dass die Spaltung der Bindung C1-C2 des Entwicklers (R) der Formel (I) in Gegenwart des Reaktanten (X) eine nachweisbare Spektralvariation hervorruft.

20. Verfahren nach Anspruch 19, **dadurch gekennzeichnet, dass** es sich bei der Wechselwirkung zwischen den Gruppen R1 oder R2 und R3 oder R4 um einen Energieübergang des FRET-Typs handelt.

21. Verfahren nach einem der Ansprüche 1 bis 10, **dadurch gekennzeichnet, dass** es sich bei dem Entwickler (R) um eine Vorstufe eines Hemmers eines Katalysators handelt sowie **dadurch**, dass die Vorstufe des Hemmers in Gegenwart des Reaktanten in einen Hemmer eines Katalysators einer nachweisbaren Reaktion umgewandelt wird, wobei sich der Katalysator von der katalytischen Aktivität, die es nachzuweisen gilt, unterscheidet.

22. Verfahren nach einem der Ansprüche 1 bis 8, **dadurch gekennzeichnet, dass** es sich bei dem Reaktanten (X) um Dansylhydrazin, das Substrat (S) oder das Produkt (P) und den Entwickler (R), dessen chemische Gruppen Keton oder Aldehyd oder Hemiacetal des Alkohol-Typs entsprechen, handelt.

23. Verfahren nach Anspruch 22, **dadurch gekennzeichnet, dass** es sich bei dem Reaktanten (X) um ein fluoreszierendes Hydrazin F-NHNH2, wobei F einem Fluorophor, der ein fluoreszierendes Produkt F-NH-N=C(Molekül) bildet, entspricht, handelt, sowie **dadurch**, dass der Reaktant (X) F-NHNH2, der nicht mit (S) oder (P) reagiert hat, mit einem Entwickler (R), der einem Entwickler-Aldehyd oder -Keton Q-C=O, wobei Q dem Quencher entspricht, entspricht, quantitativ bestimmt wird, um das nichtfluoreszierende Paar Q-CH=N-NH-F zu bilden.

24. Verfahren nach Anspruch 22, **dadurch gekennzeichnet, dass** es sich bei dem Reaktanten (X) um ein Quencher-Hydrazin des Typs Q-NH-NH2 und bei dem Entwickler (R) um ein(en) fluoreszierenden/s Aldehyd oder Keton F-CHO handelt.

25. Verfahren nach Anspruch 22, **dadurch gekennzeichnet, dass** es sich bei dem Reaktanten (X) um ein fluoreszierendes Hydrazin F1-NHNH2 und bei dem Entwickler (R) um einen Aldehyd oder ein Keton F2-CHO handelt, so dass das Auftreten eines FRET zwischen F1 und F2 beobachtet werden kann.

26. Verwendung eines Verfahrens zum Nachweisen einer katalytischen Aktivität einer Probe nach einem der vorhergehenden Ansprüche zum Nachweis einer katalytischen Umwandlung eines Substrat (S) in ein Produkt (P).
